# EUROPÄISCHE PATENTANMELDUNG

(11) **EP 2 508 195 A1**
(43) Veröffentlichungstag der Anmeldung: **10.10.2012**
(21) Anmeldenummer: 11161400.4
(22) Anmeldetag: 06.04.2011
(51) Int. Cl.: A61K 38/16, A61P 35/04

(54) **Arzneimittel enthaltend rekombinante Mistellektine zur Behandlung des malignen Melanoms**

(71) Anmelder: Cytavis BioPharma GmbH, 22525 Hamburg (DE)
(72) Erfinder: Witthohn, Klaus, 51491 Overath (DE); Lentzen, Hans, 51503 Rösrath (DE)
(74) Vertreter: Simandi, Claus

(57) **Zusammenfassung**

Die Erfindung betrifft ein Arzneimittel und / oder pharmazeutische Zusammensetzung zur Behandlung von metastasierenden Tumoren, insbesondere des malignen Melanoms, vor allem des malignen Melanom Stadium IV, und dessen Verwendung, insbesondere dessen Verwendung in ausgewählten Patientenpopulationen.

## Beschreibung

Die Erfindung betrifft ein Arzneimittel und / oder pharmazeutische Zusammensetzung zur Behandlung von metastasierenden Tumoren, insbesondere des malignen Melanoms, vor allem des malignen Melanom Stadium IV, und dessen Verwendung, insbesondere dessen Verwendung in ausgewählten Patientenpopulationen.

Das maligne Melanom (auch: schwarzer Hautkrebs) ist ein schnell und frühzeitig metastasierender Tumor der Melanozyten, Melanin-produzierende Zellen, in der basalen Zellschicht der Epidermis. Der Grad der Erkrankung ist abhängig von dem Grad der Metastasierung in die regionalen Lymphknoten und in entfernte Körperregionen. Das maligne Melanom ist besonders aggressiv und bösartig und ist verantwortlich für nahezu 80 % aller Todesfälle der Hauttumore (Parkin DM, Bray F, Ferlay J, Pisani P. 2005. CA Cancer J Clin 55: 74-108). Das maligne Melanom hat unter den Hauttumoren der Männer die größte Steigerungsrate, bei den Frauen hat es die zweitgrößte Steigerungsrate. Weltweit wird von einer Inzidenz von 160.000 neuen Fällen und 41.000 Todesfällen im Jahr ausgegangen (Parkin et al. 2005 (supra)).
Für 2010 wird in den USA mit 68.000 neuen Fällen und mit 8.700 Todesfällen gerechnet (SEER-Statistik, www.seer.cancer.gov), in Europa sind es 62.000 neue Fälle und 16.600 Todesfälle. In Australien und Neuseeland werden 10.000 neue Fälle und 1.300 Todesfälle erwartet (Parkin et al. 2005 (supra)). Für 2010 wird in den 7 bedeutenderen Pharmamärkten der Welt (USA, Japan, Frankreich, Deutschland, Italien, Spanien, Großbritannien) mit 138.000 und im Jahr 2019 mit etwa 227.000 neuen Fällen gerechnet (Globocan 2002 <http://www-dep.iarc.fr/> [Accessed April 7, 2010], World Population Prospects 2008 <http://esa.un.org/unpp/> [Accessed April 7, 2010]).

Wird das maligne Melanom im frühen Stadium diagnostiziert und behandelt, liegt die 5-Jahresüberlebensrate bei 85 %. Nach der Metastasierung (Nah- und Fernmetastasen) des Melanom sinkt die Überlebensrate drastisch. Eine prospektive Analyse von 8 klinischen Prüfungen der Eastern Cooperative Oncology Group (ECOG) mit 1362 Patienten mit metastasiertem malignen Melanom, die mit Kombichemotherapien behandelt wurden, erbrachte ein medianes Überleben von 6,4 Monaten und ein angenommenes 5-Jahresüberleben von 6%. Signifikante Parameter für ein verkürztes Überleben der Patienten mit metastasiertem Melanom sind schlechter Allgemeinzustand, viszerale Metastasen, Anzahl der betroffenen Organe und erhöhtes LDH (Lactatdehydrogenase) (Manola J, Atkins M, Ibrahim J, Kirkwood J. 2000 J Clin Oncol 18: 3782-93, Balch CM, Gershenwald JE, Soong S -J et al. 2009 J Clin Oncol 27(36): 6199 - 6206, Korn E L, Liu P-Y, Lee S J et al. 2008 J Clin Oncol 26(4): 527 - 534).

Das metastasierende maligne Melanom (sogenanntes Stadium IV) ist in der Regel eine unheilbare Erkrankung (Balch et al 2009 (supra)). Die derzeitige Standardtherapie zur Behandlung von Patienten mit metastasiertem Melanom Stadium IV ist Dacarbazine (DTIC) (Garbe C, Hauschild A, Volkenandt M et al. 2005, Deutsche Leitlinien: Malignes Melanom, www.ado-homepage.de). Dacarbazin ist gut verträglich aber es hat nur einen geringen Vorteil für die Patienten hinsichtlich der Responserate und des Überlebens. Die alleinige Anwendung von Dacarbazin erbringt eine Responserate von 5,3 % - 23%, die aber von kurzer Dauer ist (Huncharek M, Caubet J F & McGarry R. 2001 Melanoma Res 11 (1): 75 - 81, Serrone L, Zeuli M & Cognetti F 2000 J Exp Clin Res 19(1): 21 - 34). Es gibt keinen zusätzlichen Beleg durch eine klinische Prüfung der Phase III, dass Dacarbazin das Überleben der Patienten verlängert. Das mediane Überleben nach Dacarbazin in Phase III Prüfungen beträgt ca. 7,5 Monate (Chapman P B, Einhorn L H, Meyers M L et al. 1999 J Clin Oncol 17(9): 2745 - 2751, Middleton M R, Grab J J, Aaronson N et al. 2000 J Clin Oncol 18(1): 158 - 166, Atkins M B, Lotze M T, Dutcher J P et al. 1999 J Clin Oncol 17: 2105 - 2116, Falkson C 1, Ibrahim J, Kirkwood J M et al. 1998 J Clin Oncol 16: 1743 - 1751, Avril M F, Aamdal S, Grob J J et al. 2004 J Clin Oncol 22: 1118 - 1125, Flaherty L E, Atkins M, Sosman J et al. 2001 J Clin Oncol 19: 3194 - 3202). Auch andere zytotoxische Substanzen wie z.B. Temozolomid mit einer Responserate (ORR) von 13,5 - 21 %, die Substanzen Carboplatin, Cisplatin und Vindesin (ORR 12 - 26 %) und Paclitaxel und Fotemustin (ORR 7,4 - 24,2%) zeigen Aktivität bei Patienten mit metastasiertem Melanom. Die klinische Wirksamkeit dieser Therapien ist vergleichbar jener von Dacarbazin (Chapman et al. 1999 (supra), Middleton et al. 2000 (supra), Atkins et al. 1999 (supra)). So wird mit Treosulfan in einer 2.-Linien-Therapie nach Dacarbazin ein medianes Überleben von 6,5 Monaten und eine 1-Jahresüberlebensrate von 33,9 % gefunden, verbunden mit 15 - 18 % schwerwiegenden hämatologischen Nebenwirkungen (Neuber K, Reinhold U, Deutschmann A et al 2003 Melanoma Res 13: 81 - 85).

Viele dieser Substanzen werden in Kombinationstherapien angewendet (Polychemotherapie) mit dem Ziel, die Responseraten zu erhöhen und das Überleben der Patienten zu verlängern. Mit den Polychemotherapien konnte zwar die Responserate erhöht werden, jedoch hatte die Therapie keinen Einfluss auf die Überlebensrate (OS) im Vergleich zur alleinigen Anwendung von Dacarbazin (Agarwala S S, Glaspy J, O'Day S J et al. 2002 J Clin Oncol 20: 125 - 133, Eton O, Legha S S, Bedikian A Y et al. 2002 J Clin Oncol 20: 2045 - 2052, Falkson et al 1998 (supra), Avril et al. 2004 (supra)). Zwei Beispiele der Polychemotherapien sind das BHD-Regime (ORR: 12.7 % - 30.4 %) und das DVP-Regime (ORR: 31.4 % - 45 %).

Zusätzlich zur Chemotherapie wird seit einigen Jahren bei Patienten mit metastasiertem malignen Melanom eine Immuntherapie mit dem für diese Therapie zugelassener HochDosis Interleukin-2 (IL-2) angewendet. Berichte mit signifikanten klinischen Effekten sind bekannt, obwohl ausgewählte Patientengruppen betroffen sind (Danson S, Lorigan P, Arance A et al. 2003 J Clin Oncol 21: 2551 - 2557). Die erreichten Tumor-Responseraten (ORR: 16% - 21.5%), sind jedoch begleitet von extensiven Multiorgantoxizitäten und limitieren somit die Anwendung von IL-2. Ähnlich verhält es sich bei der Anwendung von Hochdosis und mittlerer Dosis von Interferon-α (IFN-α). Die Behandlung mit GM-CSF schien nur in kleinen Studien und in klinischen Prüfungen der frühen Phase erfolgreich zu sein.

Die Kombination von Chemotherapeutika und Zytokinen (Polychemoimmuntherapie) zeigt partiell höhere Responseraten (ORR) im Vergleich zu den Monotherapien, aber erbringt keine Verbesserung des Überlebens. So zeigte die Kombination von IL-2 und Cisplatin mit 50 % eine hohe Responserate kurzer Dauer, die jedoch von starken Nebenwirkungen (unerwünschten Wirkungen) begleitet waren. Ein Vergleich einer Monotherapie mit Dacarbazin und einer Kombination von Dacarbazin, Cisplatin, IFN-α und IL-2 zeigte keinen Unterschied zwischen den beiden Behandlungsarmen (Flaherty et al. 2001 (supra), Danson et al. 2003 (supra), Agarwala et al. 2002 (supra), Eton et al 2002 (supra), Falkson et al 1998 (supra)). Eine Kombination von Chemotherapie (Cisplatin, Vinblastine, Dacarbazin) verbunden mit einer Daueranwendung von Biotherapeutika (Interleukin-2, Interferon alfa-2b und GM-CSF in verschiedenen Regimen) zeigte für Patienten mit metastasiertem malignen Melanom ein medianes Überleben von 14 Monaten. Diese Verlängerung des allgemeinen Überlebens war begleitet von einer großen Anzahl an hämatologischen und nichthämatologischen Nebenwirkungen mit CTC Grad 3 und CTC Grad 4. (O'Day S J, Atkins M B, Boasberg P et al. 2009 J Clin Oncol 27(36): 6207 - 6212).

Betreffend metastasierender Tumore sind die Studienergebnisse von Ipilimumab (BMS, Yervoy®), einem monoklonalen Antikörper, der humanes CTLA-4 erkennt, erfolgreich. Unter Therapie mit Ipilimumab konnte das mediane Überleben der Patienten mit metastasiertem Melanom (Stadium III und Stadium IV) signifikant verlängert werden und zwar auf 10 Monate versus 6,4 Monate in der Kontrollgruppe (Hodi F S, O'Day S J, McDermott D F et al. 2010 N Engl J Med 363 (8): 711 - 723). Dies entspricht einer 1-Jahresüberlebensrate von 45,6 % in der Ipilimumab Gruppe im Vergleich zu 25,3 % in der Kontrollgruppe. Die Nebenwirkungen unter Ipilimumab werden jedoch als sehr schwerwiegend betrachtet. 10 - 15 % der Patienten hatten schwerwiegende immun-bedingte Nebenwirkungen (CTC-Grad 3 und Grad 4) mit Auswirkungen auf der Haut und auf den Darmtrakt (Hodi et al. 2010 (supra)).

Es besteht daher ein großes Bedürfnis Arzneimittel bereitzustellen, die Patientenpopulationen im Stadium III oder IV mit metastasierenden Tumoren besser versorgen oder zumindest die Lebenserwartung signifikant erhöhen, insbesondere beim Versagen einer Standardtherapie.

Mistelextrakte werden seit Jahrhunderten therapeutisch genutzt. Insbesondere in der Krebstherapie sind Mistelpräparate mit unterschiedlichem Erfolg eingesetzt worden (Bocci V 1993 J Biol Regulators and Homeostatic Agents 7(1): 1 - 6; Gabius H-J, Gabius S, Joshi S S et al. 1993 Planta Med 60: 2 - 7; Gabius H-J & Gabius S 1994 PZ 139: 9 - 16; Ganguly C & Das S 1994 Chemotherapy 40: 272 - 278, Hajto T, Hostanska K, Gabius H_J 1989 Cancer Res 49: 4803 - 4808, Hajto T, Hostanska K, Frei K et al. 1990 Cancer Res. 50: 3322 - 3326). Es zeigte sich, dass die therapeutischen Effekte insbesondere durch sogenannte Mistellektine (Viscumine, Viscum album Agglutinine, VAA) vermittelt werden. Den Mistellektinen wird dabei neben einer zytotoxischen Wirkung auch eine unspezifische Immunstimulation zugesprochen, deren positive Effekte zur Therapie von Tumorpatienten genutzt werden. Verschiedene Untersuchungen mit Mistellektinen in vitro (Hajto et al., 1990 (supra); Männel D N, Becker H, Gundt A et al. 1991 Cancer Immunol Immunother 33: 177 - 182; Beuth J, Ko K L, Tunggal L et al. 1993 Drug Res 43: 166 - 169) und in vivo (Hajto T 1986 Oncology 43 suppl 1: 51 - 65; Hajto et al., 1989 (supra), Beuth J, Ko H L, Gabius H-J et al. 1991 In Vivo 5: 29 - 32; Beuth J, Ko H L, Gabius H-J et al. 1992 J Clin Invest 70: 658 - 661), sowie klinische Studien (Beuth et al., 1992 (supra)) zeigten eine erhöhte Freisetzung von inflammmatorischen Zytokinen (TNF-alpha, IL-1, IL-6) und eine Aktivierung von zellulären Komponenten des Immunsystems (TH -Zellen, NK-Zellen).

Durch Analysen des Mistelextraktes konnten bisher drei Mistellektine (ML-1, ML-II, ML-III) mit unterschiedlichen Molekulargewichten und Zuckerbindungsspezifitäten identifiziert werden. Es konnte gezeigt werden, dass der immunstimulierende Effekt des Mistelextrakts auf das ML-I zurückzuführen ist. Das ML-I-Lektin besteht aus jeweils zwei glykosylierten A- und B-Ketten (MLA bzw. MLB). Die A-Kette ist für eine enzymatische Inaktivierung von Ribosomen (Endo Y, Tsurugi K & Franz H 1988 FEBS Lett 231: 378 - 380) verantwortlich ist, während die B-Kette bei der Carbohydratbindung beteiligt ist. Die beiden Ketten sind durch Disulfidbrücken miteinander verknüpft. Die resultierenden Mistellektin-Monomere können sich unter Ausbildung von nicht kovalenten Bindungen zu Dimeren zusammenlagern.

Es ist möglich, das biologisch aktive Mistellektin auch rekombinant herzustellen. EP 0751221 beschreibt die Reindarstellung von Mistellektin-Polypeptiden als strukturell homogene Substanz, wobei ausgehend von den Gensequenzen des Mistellektins rekombinante, hochreine Einzelketten (A-Kette, B-Kette) hergestellt werden, die *in vitro* reassoziiert werden können und so ein rekombinantes Mistellektin-Holoprotein ergeben, das proteinchemisch, enzymatisch und strukturell homogen ist, sogenanntes Aviscuminum. Gemäß EP 0751221 ist das rekombinante Mistellektin-Polypeptid sowohl als Holoprotein, als Teilkette und in Form von Subfragmenten für therapeutische Zwecke geeignet und erfindungsgemäß umfasst.

Bisher wurden rekombinante Mistellektine insbesondere in der Behandlung von Tumorerkrankungen eingesetzt. Die Verwendung von rekombinanten Mistellektinen zur Behandlung von Hautkrebs, insbesondere eines malignen Melanoms auch in der Form eines metastasierenden Tumors ist jedoch nicht im Stand der Technik beschrieben.

Überraschenderweise konnte nunmehr gezeigt werden, dass unter der Therapie mit rekombinanten Mistellektinen das Überleben von Tumorpatienten mit metastasierendem Melanom (Stadium III und IV) signifikant verlängert werden kann bzw. die 1-JahresÜberlebensrate signifikant steigt.

Die Aufgabe der vorliegenden Erfindung besteht darin, ein Arzneimittel und pharmazeutisches Mittel bereitzustellen mit denen ein metastasierender Tumor, vorzugsweise Hautkrebs, insbesondere malignes Melanom an Tier, Säugetier und Mensch wirksam behandelt werden kann.

Die Aufgabe wird durch die Bereitstellung eines Arzneimittels sowie einer pharmazeutischen Zusammensetzung gelöst, wobei diese rekombinante Mistellektine enthalten zur Behandlung von metastasierenden Tumoren, vorzugsweise Hautkrebs, insbesondere malignes Melanom, wobei die rekombinanten Mistellektine die folgenden Aminosäuresequenzen umfassen. Vorzugsweise umfasst das erfindungsgemäße Arzneimittel die Mistellektin-A-Kette (MLA) beziehungsweise die Mistellektin-B-Kette (MLB), jeweils einzeln oder zusammen, auch in Form von Dimeren (Siehe z.B. EP 0 751 221 oder EP 1 051 495).

Das rekombinante Mistellektin-Polypeptid der Mistellektin-A Kette umfasst die folgenden Sequenzen: SEQ ID No. 1 - 3, einschließlich deren Isoformen oder ein funktionelles Fragment davon.

Das rekombinante Mistellektin-Polypeptid der Mistellektin-B Kette umfasst die folgenden Sequenzen SEQ ID No. 4 - 12, einschließlich deren Isoformen oder ein funktionelles Fragment davon.

### (nachstehend allesamt "rekombinante Mistellektine")

Weiterhin bevorzugt ist ein erfindungsgemäßes rekombinantes Mistellektin ein Heterodimer bestehend aus den Sequenzen SEQ ID No. 1 und SEQ ID No. 4, siehe z.B. EP 0 751 221.

Der Begriff "funktionelles Fragment" definiert im Zusammenhang mit dieser Erfindung Fragmente der genannten Polypeptide, welche die gleiche biologische Funktion besitzen, wie das mit der jeweiligen Aminosäuresequenz oben dargestellte Polypeptid.

Der Begriff "gleiche biologische Funktion" beschreibt in diesem Zusammenhang beispielsweise, dass Fragmente oder Derivate der Polypeptide die gleichen Signale in einer Zelle induzieren, wie die genannten Peptide. Beispiele für Fragmente sind Peptiddomänen mit definierten Funktionen. Die "gleiche biologische Funktion" umfasst auch die Zytotoxizität, Immunstimmulation (sowohl des nativen, als auch des adaptiven Immunsysterns), Stimulation der Freisetzung von Zytokinen, Antigenität, die Induktion der Expression oder die Aktivierung von Oberflächenmarkern, die Induktion von Apoptose oder Endorphin-Stimulation.

Unter "biologischer Aktivität des rekombinanten Mistellektins" wird hier jede biologische Aktivität aus dem Spektrum der gesamten biologischen Aktivitäten des rekombinanten Mistellektins verstanden. Eine derartige Funktion ist z.B. die pharmakologische Wirkung des rekombinanten Mistellektins.

Untersuchungen von ML-I-Monomeren ergaben 25 verschiedene Isoformen, die auf unterschiedliche Kombinationen verschiedener A- und B-Ketten sowie unterschiedliche Glykosylierungszustände der Ketten zurückzuführen sind.

Für die vorliegende Erfindung kommt deshalb auch ein Mistellektin-Polypeptid oder ein Fragment davon, welches die Sequenzvariabilität der verschiedenen MLA- und MLB-Ketten umfasst, zu den Sequenzen SEQ ID No. 1 - 12 erfindungsgemäß in Betracht.

Vorzugsweise enthält das erfindungsgemäße Arzneimittel eines rekombinanten Mistellektin-Polypeptids mit Sequenzen SEQ ID No. 1 - 12 oder einem funktionellen Fragment davon oder eine beliebige Kombination davon.

Bevorzugt ist weiterhin, dass die Verwendung von erfindungsgemäßen rekombinanten Mistellektinen bei solchen Patientenpopulationen zum Tragen kommt, die mittels einer Standardtherapie nicht auf Tumorpräparate ansprechen, bzw. Nicht-Responder oder Therapieversager umfassen.

Daher umfasst die Erfindung solche Patienten oder Patientenpopulationen von Nicht-Respondern und Therapieversagern bei metastasierenden Tumoren, insbesondere maligne Melanome und Hautkrebs, besonders bevorzugt im Stadium III und IV, bei denen eine Standardtumortherapie nicht erfolgreich ist.

Daher betrifft die Erfindung eine solche Auswahl von Patienten oder Patientenpopulationen, die nach erster Behandlung von Tumoren, insbesondere malignen Melanomen und Hautkrebs, mit einem Tumorpräparat, wie oben beispielhaft für das maligne Melanom beschrieben, mit den erfindungsgemäßen rekombinanten Mistellektinen behandelt werden. Daher werden bevorzugt solche Patienten oder Patientenpopulationen mit den erfindungsgemäßen rekombinanten Mistellektinen behandelt, die im fortgeschrittenen oder Endstadium einer Tumorerkrankung stehen, wobei eine Metastasierung (Stadium III und IV), insbesondere bei einem malignen Melanom, eingetreten ist. Von daher ist beispielsweise zu einem malignem Melanom ebenfalls eine Kombinationstherapie eines Patienten erfindungsgemäß umfasst, wobei zuerst ein erstes Anti-Tumormittel, wie beispielsweise Dacarbazin, Dacarbazin kombiniert mit Interferon-α, Dacarbazin kombiniert mit Vindesin, Treosulfan kombiniert mit Gemcitabin, Imatinib verabreicht wird, und anschließend alleine oder in Kombination zusätzlich die erfindungsgemäßen rekombinanten Mistellektinen verabreicht werden.

Besonders bevorzugt ist das erfindungsgemäße Arzneimittel zur Behandlung von malignen Melanomen im Stadium III und IV geeignet, da überraschender Weise eine signifikante Lebensverlängerung eines einzelnen Patienten oder einer entsprechenden Patientenpopulation erreicht werden kann.

Dieses Ergebnis ist völlig unerwartet und diese spezielle Eignung und Vorteil kann von einem Tumorarzneimittel per se nicht erwartet werden.

Von daher betrifft das Arzneimittel ein neues Anti-Tumormittel und zwar zur Behandlung von metastasierenden Tumoren, insbesondere von malignen Melanomen, vorzugsweise im Stadium III und IV.

Im Sinne dieser Erfindung ist ein "malignes Melanom" wie vorweg beschrieben zu verstehen, wobei jedoch die Stadien III und IV solche Formen eines malignen Melanoms beschreiben, die erfindungsgemäß eine Metastasierung des Tumors darlegen (siehe z.B. Beschreibung im Pschyrembel ®, De Gruyter Verlag, Berlin).

Die Erfindung betrifft außerdem ein Arzneimittel für die Behandlung des malignen Melanoms, welches das rekombinante Mistellektin-Polypeptid, gegebenenfalls zusammen mit einem pharmazeutisch verträglichen Träger enthält unter Ausbildung einer pharmazeutischen Zusammensetzung. Beispiele für besonders geeignete pharmakologisch verträgliche Träger sind dem Fachmann auf dem Gebiet der Tumorheilkunde bekannt und umfassen gepufferte Kochsalzlösungen, Wasser, u.a., verschiedene Arten von Detergenzien, sterile Lösungen, etc. Arzneimittel, die solche Träger umfassen, können mittels bekannter konventioneller Methoden formuliert werden. Diese Arzneimittel können einem Individuum in einer geeigneten Dosis verabreicht werden. Die Verabreichung kann lokal, oral oder parenteral erfolgen, z.B. intravenös, intraperitoneal, subcutan, intramuskulär, lokal, intranasal, intrabronchial oder intradermal, oder über einen Katheter an einer Stelle in einer Arterie. Die Art der Dosierung wird vom behandelnden Arzt entsprechend den klinischen Faktoren bestimmt. Es ist dem Fachmann bekannt, dass die Art der Dosierung von verschiedenen Faktoren abhängig ist, wie z.B. der Körpergröße bzw. dem Gewicht, der Körperoberfläche, dem Alter, dem Geschlecht oder der allgemeinen Gesundheit des Patienten, aber auch von dem speziell zu verabreichenden Mittel, der Dauer und Art der Verabreichung, und von anderen Medikamenten, die möglicherweise parallel verabreicht werden.

Die pharmazeutische Zusammensetzung, welche die erfindungsgemäßen rekombinanten Mistellektin-Polypeptide umfasst, kann lokal oder systemisch verabreicht werden.

Die pharmazeutische Zusammensetzung wird erfindungsgemäß bei der Behandlung des malignen Melanoms verwendet.

Als vorteilhaft hat sich eine Dosierung der erfindungsgemäßen Mistellektine für die humane Anwendung von 2-10 ng/kg (Körpergewicht) erwiesen. Besonders vorteilhaft ist die Dosierung einem Bereich von 3 - 7 ng/kg. Vorzugsweise beträgt die verabreichte Menge 5 ng/kg Körpergewicht. Die bevorzugte nicht auf das Körpergewicht bezogene humane Dosierung beträgt 350 ng.

Das erfindungsgemäße Arzneimittel wird über eine Zeitdauer von mindestens 8 Wochen mit einer Frequenz von 1 x täglich bis 1 x pro Woche appliziert. Vorzugsweise wird das Arzneimittel 2 bis 3 x pro Woche verabreicht, besonders bevorzugt ist 2 x pro Woche.

Daher betrifft die Erfindung ein Verfahren zur Dosierung der erfindungsgemäßen rekombinanten Mistellektine oder des erfindungsgemäßen Arzneimittels, wobei die Dosierung 2 bis 10 ng/kg (Körpergewicht) beträgt. Insbesondere betrifft die Erfindung ein Verfahren zur Dosierung der erfindungsgemäßen rekombinanten Mistellektine oder des erfindungsgemäßen Arzneimittels, wobei die Dosis 200 - 500 ng, inbesondere 350 ng beträgt und mindestens 1 x pro Woche dem Patienten verabreicht wird. Der Patient ist bevorzugt ein Patient im fortgeschrittenen oder Endstadium einer Tumorerkrankung, wobei eine Metastasierung (Stadium III und IV), insbesondere bei einem malignen Melanom, eingetreten ist.

Nachfolgende Beispiele und Figuren dienen zur Erläuterung der Erfindung, ohne jedoch die Erfindung auf diese Beispiele zu beschränken.

### Beispiele und Figuren:

### Beispiel 1 einer Zusammensetzung des Arzneimittels

Lösung zur Injektion: 1 mL Ampulle mit 0,5 mL / 1,0 mL Injektionslösung

| | | |
|---|---|---|
| Aviscuminum | 200 - 500 ng | |
| Natriummonohydrogenphosphat Dihydrat | 2,8 mg | 5,6 mg |
| Natriumdihydrogenphosphat Dihydrat | 0,078 mg | 0,155 mg |
| Natriumchlorid | 3,3 mg | 6,7 mg |
| Polyoxyethylen sorbitan ester (Polysorbat) | 0,1 mg | 0,1 mg |
| Glutaminsäure | 0,1 mg | 0,1 mg |
| Wasser zur Injektion | ad 0,5 ml | ad 1,0 mL |

### Beispiel 2 einer Zusammensetzung des Arzneimittels

Pulver zur Herstellung einer Lösung zur Injektion, 2R Glasvial mit

| | |
|---|---|
| Aviscuminum | 200 - 500 ng |

| | |
|---|---|
| Trehalose | 40,0 mg |
| Natriumchlorid | 1,0 mg |
| Tris(hydroxymethyl)aminomethan (TRIS) | 0,6 mg |
| Polyoxyethylen sorbitan ester (Polysorbat) | 0,1 mg |
| Salzsäure zur Einstellung des pH Werts | |

zur Verabreichung wird das Pulver mit 0,5 mL oder 1,0mL Wasser zur Injektion gelöst.

Im Rahmen einer klinischen Studie sollte nun geprüft werden, ob rekombinantes Mistellektin (rML gemäß EP 0 751 221) bei Patienten mit metastasierendem malignen Melanom Stadium IV nach Versagen der Standardtherapie das Fortschreiten der Erkrankung aufhalten kann und ob das Überleben der Patienten verlängert werden kann. In die Studie eingeschlossen wurden 31 auswertbare Patienten. Obwohl auch eine Veränderung des progressionsfreien Überlebens eintrat, wurde überraschenderweise das Überleben der Patienten signifikant erhöht. Das mediane Überleben der Patienten betrug 11,0 Monate, die 1-Jahresüberlebensrate betrug 45,0 %. Die Verlängerung des Überlebens war unabhängig von der Anzahl der Vortherapien und unabhängig vom Allgemeinbefinden (ECOG-Status 0 oder 1). Die 1-Jahresüberlebensrate einer vergleichbaren Kontrollgruppe, die entsprechend den Kriterien Geschlecht, Hirnmetastasen vorhanden / nicht vorhanden, Art der Metastasen (viszeral / nicht viszeral) und Allgemeinbefinden (ECOG) nach den Daten von Korn et al. 2008 (supra) kalkuliert werden kann, beträgt 33,1 %. Es traten keine Nebenwirkungen mit einen Schweregrad von >2 entsprechend den CTC-Kriterien unter der Therapie von rML auf. Damit ist die Anwendung von rML sehr gut verträglich.

**Tabelle 1:**

| Demographische Daten (Fachbegriffe in Englisch) | | | |
|---|---|---|---|
| | | | |
| Patients, n=31 | | | |
| Sex | n (%) | Male | 16 (51.6) |
| | | female | 15 (48.4) |
| ECOG | n (%) | 0 | 17 (54.8) |
| | | 1 | 14 (45.2) |
| Age | Mean | | 65.32 |
| (yrs) | SD | | 13.53 |
| | Median | | 67.00 |
| Range | | | 20 - 86 |
| Weight (kg) | Mean | | 76.53 |
| | SD | | 12.42 |
| | Median | | 77.50 |
| Type of melanoma | n (%) | cutaneous | 26 (83.9) |
| | | mucosal | 3 (9.7) |
| | | occult | 1 (3.2) |
| | | other | 1 (3.2) |
| No. of metastatic sites | n (%) | 1 | 13 (41.9) |
| | | 2 | 13 (41.9) |
| | | 3 | 4 (12.9) |
| | | 4 | 1 (3.2) |
| LDH (U/L) at BL | Mean | | 262.71 |
| | SD | | 89.17 |
| | Median | | 245.00 |
| LDH elevation | n (%) | yes | 17 (54.8) |
| | | no | 14 (45.2) |

| | | | |
|---|---|---|---|
| ECOG = Eastern Cooperative Oncology Group, LDH = Lactatdehydrogenase | | | |

### Fallbeispiel 1:

Patient, weiblich, Alter: 78 Jahre, malignes Melanom Stadium IV, ECOG: 1,
Metastasen in Lymphknoten und Lunge,
2 Vorbehandlungen mit Dacarbazin,
15 Zyklen (420 Tage) Therapie mit Aviscuminum (rML) 350 ng, 2 x pro Woche,
Stabilisierung der Erkrankung (kein Tumorwachstum) über einen Zeitraum von 433 Tagen,
Überleben: 453 Tage

### Fallbeispiel 2:

Patient, männlich, Alter: 79 Jahre, malignes Melanom Stadium IV, ECOG: 0,
multiple Metastasen in Leber und Lunge,

5 Vorbehandlungen mit Dacarbazin, Dacarbazin kombiniert mit Interferon-α, Dacarbazin
kombiniert mit Vindesin, Treosulfan kombiniert mit Gemcitabin, Imatinib
4 Zyklen (112 Tage) Therapie mit Aviscuminum (rML) 350 ng, 2 x pro Woche,
Stabilisierung der Erkrankung (kein Tumorwachstum) über einen Zeitraum von 116 Tagen,
Überleben: 435 Tage

### Beschreibung der Figuren:

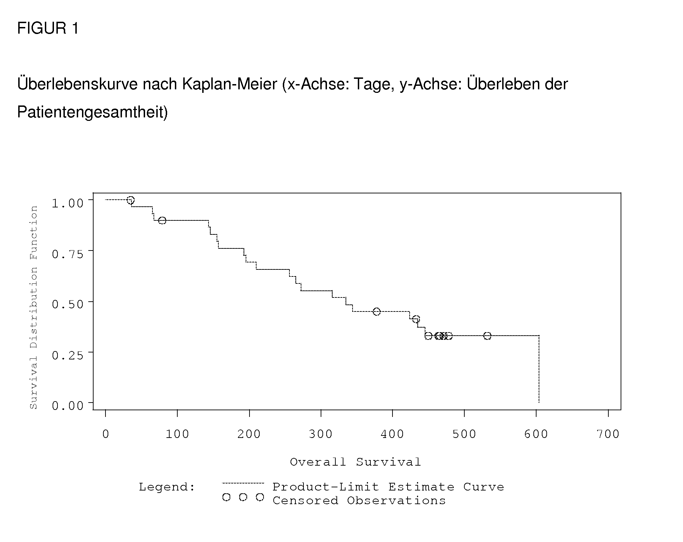

Figur 1 beschreibt die zu den Studiendaten ausgewertete Überlebenskurve nach der Methode von Kaplan-Meier.

## Patentansprüche

1. Arzneimittel enthaltend rekombinantes Mistellektin zur Behandlung von metastasierenden Tumoren oder Hautkrebs, wobei das rekombinante Mistellektin ausgewählt ist aus der Gruppe der Aminosäuresequenzen SEQ ID No. 1 -12 oder Teile und Fragmente davon umfasst oder eine Kombination davon.

2. Arzneimittel nach Anspruch 1, wobei das rekombinante Mistellektin-Polypeptid eine Mistellektin-A-Kette ist, ausgewählt aus der Gruppe der Aminosäuresequenzen SEQ ID No. 1 - 3 oder Teile und Fragmente davon umfasst oder eine Kombination davon.

3. Arzneimittel nach Anspruch 1, wobei das rekombinante Mistellektin-Polypeptid eine Mistellektin-B-Kette ist, ausgewählt aus der Gruppe der Aminosäuresequenzen SEQ ID No. 4 - 12 oder Teile und Fragmente davon umfasst oder eine Kombination davon.

4. Arzneimittel nach einem der Ansprüche 1 bis 3, wobei der metastasierende Tumor oder Hautkrebs ein malignes Melanom ist.

5. Arzneimittel nach einem der Ansprüche 1 bis 4, wobei das Arzneimittel ausgewählt ist für Nicht-Responder und Therapieversager einer Standardtumortherapie.

6. Arzneimittel nach einem der Ansprüche 1 bis 5, wobei das Arzneimittel ausgewählt ist für die Behandlung der Stadien III und IV eines metastasierenden Tumors oder Hautkrebs, insbesondere eines malignen Melanom.

7. Arzneimittel nach einem der Ansprüche 1 bis 6, wobei das Arzneimittel nach einer ersten Tumorstandardtherapie verwendet wird.

8. Arzneimittel enthaltend ein rekombinantes Mistellektin-Polypeptid gemäß einem der Ansprüche 1 bis 7, gegebenenfalls zusammen mit einem pharmazeutisch verträglichen Träger.

9. Pharmazeutische Zusammensetzung enthaltend mindestens ein rekombinantes Mistellektin-Polypeptid gemäß einem der Ansprüche 1 bis 8 umfasst, zusammen mit einem pharmazeutisch verträglichen Träger oder ggfs. weiteren Hilfs- und Zusatzstoffen.

10. Verfahren zur Dosierung einer pharmazeutische Zusammensetzung enthaltend mindestens ein rekombinantes Mistellektin-Polypeptid gemäß Anspruch 9, **dadurch gekennzeichnet, dass** die Dosierung 2 bis 10 ng/kg (Körpergewicht) beträgt.


SEQUENCE LISTING

<110>  Cytavis Biopharma GmbH

<120>  Arzneimittel enthaltend rekombinante Mistellektine zur Behandlung des
malignen Melanoms
<130>  CYT22

<160>  12

<170>  PatentIn version 3.3

<210>  1
<211>  253
<212>  PRT
<213>  Artificial

<220>
<223>  EP0751221 recombinant Protein


<220>
<221>  misc_feature
<222>  (1)..(1)
<223>  Xaa can be Met or can be deleted

<220>
<221>  misc_feature
<222>  (5)..(5)
<223>  Xaa can be Ile or Leu

<220>
<221>  misc_feature
<222>  (16)..(16)
<223>  Xaa can be Glu or Asp

<400>  1

Xaa Tyr Glu Arg Xaa Arg Leu Arg Val Thr His Gln Thr Thr Gly Xaa
1               5                   10                  15


Glu Tyr Phe Arg Phe Ile Thr Leu Leu Arg Asp Tyr Val Ser Ser Gly
            20                  25                  30


Ser Phe Ser Asn Glu Ile Pro Leu Leu Arg Gln Ser Thr Ile Pro Val
        35                  40                  45


Ser Asp Ala Gln Arg Phe Val Leu Val Glu Leu Thr Asn Gln Gly Gly
    50                  55                  60


Asp Ser Ile Thr Ala Ala Ile Asp Val Thr Asn Leu Tyr Val Val Ala
65                  70                  75                  80


Tyr Gln Ala Gly Asp Gln Ser Tyr Phe Leu Arg Asp Ala Pro Arg Gly
                85                  90                  95


Ala Glu Thr His Leu Phe Thr Gly Thr Thr Arg Ser Ser Leu Pro Phe
            100                 105                 110


Asn Gly Ser Tyr Pro Asp Leu Glu Arg Tyr Ala Gly His Arg Asp Gln
        115                 120                 125

Ile Pro Leu Gly Ile Asp Gln Leu Ile Gln Ser Val Thr Ala Leu Arg
    130                 135                 140

Phe Pro Gly Gly Ser Thr Arg Thr Gln Ala Arg Ser Ile Leu Ile Leu
145                 150                 155                 160

Ile Gln Met Ile Ser Glu Ala Ala Arg Phe Asn Pro Ile Leu Trp Arg
                165                 170                 175

Ala Arg Gln Tyr Ile Asn Ser Gly Ala Ser Phe Leu Pro Asp Val Tyr
            180                 185                 190

Met Leu Glu Leu Glu Thr Ser Trp Gly Gln Gln Ser Thr Gln Val Gln
        195                 200                 205

His Ser Thr Asp Gly Val Phe Asn Asn Pro Ile Arg Leu Ala Ile Pro
    210                 215                 220

Pro Gly Asn Phe Val Thr Leu Thr Asn Val Arg Asp Val Ile Ala Ser
225                 230                 235                 240

Leu Ala Ile Met Leu Phe Val Cys Gly Glu Arg Pro Ser
                245                 250

<210>  2
<211>  256
<212>  PRT
<213>  Artificial

<220>
<223>  EP1051495 recombinant Protein

<220>
<221>  misc_feature
<222>  (1)..(1)
<223>  Xaa can be Met or can be deleted

<220>
<221>  misc_feature
<222>  (16)..(16)
<223>  Xaa can be Asp or Glu
<220>
<221>  misc_feature
<222>  (64)..(64)
<223>  Xaa can be Gly or Gln
<220>
<221>  misc_feature
<222>  (67)..(67)
<223>  Xaa can be Ile or Val

<220>
<221>  misc_feature
<222>  (76)..(76)
<223>  Xaa can be Leu or Ala
<220>
<221>  misc_feature
<222>  (108)..(108)

<223>  Xaa can be Asp-Arg or can be deleted

<220>
<221>  misc_feature
<222>  (114)..(114)
<223>  Xaa can be Asn or Thr

<220>
<221>  misc_feature
<222>  (118)..(118)
<223>  Xaa can be Pro or Thr

<220>
<221>  misc_feature
<222>  (135)..(135)
<223>  Xaa can be Asp or Glu

<220>
<221>  misc_feature
<222>  (142)..(142)
<223>  Xaa can be Ser or Thr

<220>
<221>  misc_feature
<222>  (146)..(146)
<223>  Xaa can be Phe or Tyr

<220>
<221>  misc_feature
<222>  (153)..(153)
<223>  Xaa can be Ala or Thr

<220>
<221>  misc_feature
<222>  (178)..(178)
<223>  Xaa can be Ala or Tyr
<220>
<221>  misc_feature
<222>  (181)..(181)
<223>  Xaa can be Tyr or Asp

<220>
<221>  misc_feature
<222>  (186)..(186)
<223>  Xaa can be Ala or Glu

<220>
<221>  misc_feature
<222>  (192)..(192)
<223>  Xaa can be Val or Met
<220>
<221>  misc_feature
<222>  (220)..(220)
<223>  Xaa can be Ile or Phe

<220>
<221>  misc_feature
<222>  (225)..(226)
<223>  Xaa can be Pro-Ser or Pro-Thr

<220>
<221>  misc_feature
<222>  (233)..(233)
<223>  Xaa can be Thr or Ser

<220>
<221>  misc_feature
<222>  (237)..(237)

<223>  Xaa can be Asp or Ser

<220>
<221>  misc_feature
<222>  (255)..(256)
<223>  Xaa can be Ser-Ser or can be deleted

<400>  2

Xaa Tyr Glu Arg Leu Arg Leu Arg Val Thr His Gln Thr Thr Gly Xaa
1               5                   10                  15

Glu Tyr Phe Arg Phe Ile Thr Leu Leu Arg Asp Tyr Val Ser Ser Gly
            20                  25                  30

Ser Phe Ser Asn Glu Ile Pro Leu Leu Arg Gln Ser Thr Ile Pro Val
        35                  40                  45

Ser Asp Ala Gln Arg Phe Val Leu Val Glu Leu Thr Asn Gln Gly Xaa
    50                  55                  60

Asp Ser Xaa Thr Ala Ala Ile Asp Val Thr Asn Xaa Tyr Val Val Ala
65                  70                  75                  80

Tyr Gln Ala Gly Asp Gln Ser Tyr Phe Leu Arg Asp Ala Pro Arg Gly
                85                  90                  95

Ala Glu Thr His Leu Phe Thr Gly Thr Thr Arg Xaa Ser Ser Leu Pro
            100                 105                 110

Phe Xaa Gly Ser Tyr Xaa Asp Leu Glu Arg Tyr Ala Gly His Arg Asp
        115                 120                 125

Gln Ile Pro Leu Gly Ile Xaa Gln Leu Ile Gln Ser Val Xaa Ala Leu
    130                 135                 140

Arg Xaa Pro Gly Gly Ser Thr Arg Xaa Gln Ala Arg Ser Ile Leu Ile
145                 150                 155                 160

Leu Ile Gln Met Ile Ser Glu Ala Ala Arg Phe Asn Pro Ile Leu Trp
                165                 170                 175

Arg Xaa Arg Gln Xaa Ile Asn Ser Gly Xaa Ser Phe Leu Pro Asp Xaa
            180                 185                 190

Tyr Met Leu Glu Leu Glu Thr Ser Trp Gly Gln Gln Ser Thr Gln Val
        195                 200                 205

Gln His Ser Thr Asp Gly Val Phe Asn Asn Pro Xaa Arg Leu Ala Ile
    210                 215                 220

Xaa Xaa Gly Asn Phe Val Thr Leu Xaa Asn Val Arg Xaa Val Ile Ala
225                 230                 235                 240

Ser Leu Ala Ile Met Leu Phe Val Cys Gly Glu Arg Pro Ser Xaa Xaa
                245                 250                 255

<210>  3
<211>  257
<212>  PRT
<213>  Artificial

<220>
<223>  EP1051495 recombinant Protein

<220>
<221>  misc_feature
<222>  (1)..(1)
<223>  Xaa can be Met or can be deleted

<400>  3

Xaa Tyr Glu Arg Leu Arg Leu Arg Val Thr His Gln Thr Thr Gly Asp
1               5                   10                  15

Glu Tyr Phe Arg Phe Ile Thr Leu Leu Arg Asp Tyr Val Ser Ser Gly
            20                  25                  30

Ser Phe Ser Asn Glu Ile Pro Leu Leu Arg Gln Ser Thr Ile Pro Val
        35                  40                  45

Ser Asp Ala Gln Arg Phe Val Leu Val Glu Leu Thr Asn Gln Gly Gln
    50                  55                  60

Asp Ser Ile Thr Ala Ala Ile Asp Val Thr Asn Ala Tyr Val Val Ala
65                  70                  75                  80

Tyr Gln Ala Gly Asp Gln Ser Tyr Phe Leu Arg Asp Ala Pro Arg Gly
                85                  90                  95

Ala Glu Thr His Leu Phe Thr Gly Thr Thr Arg Asp Arg Ser Ser Leu
            100                 105                 110

Pro Phe Thr Gly Ser Tyr Thr Asp Leu Glu Arg Tyr Ala Gly His Arg
        115                 120                 125

Asp Gln Ile Pro Leu Gly Ile Glu Gln Leu Ile Gln Ser Val Ser Ala
    130                 135                 140

Leu Arg Tyr Pro Gly Gly Ser Thr Arg Ala Gln Ala Arg Ser Ile Leu
145                 150                 155                 160

Ile Leu Ile Gln Met Ile Ser Glu Ala Ala Arg Phe Asn Pro Ile Leu
                165                 170                 175

Trp Arg Tyr Arg Gln Asp Ile Asn Ser Gly Glu Ser Phe Leu Pro Asp
            180                 185                 190

Met Tyr Met Leu Glu Leu Glu Thr Ser Trp Gly Gln Gln Ser Thr Gln
        195                 200                 205

Val Gln His Ser Thr Asp Gly Val Phe Asn Asn Pro Phe Arg Leu Ala
    210                 215                 220

Ile Ser Thr Gly Asn Phe Val Thr Leu Ser Asn Val Arg Ser Val Ile
225                 230                 235                 240

Ala Ser Leu Ala Ile Met Leu Phe Val Cys Gly Glu Arg Pro Ser Ser
                245                 250                 255

Ser

<210> 4
<211> 264
<212> PRT
<213> Artificial

<220>
<223> EP0751221 recombinant Protein

<220>
<221> misc_feature
<222> (1)..(1)
<223> Xaa can be Met or can be deleted

<400> 4

Xaa Asp Asp Val Thr Cys Ser Ala Ser Glu Pro Thr Val Arg Ile Val
1               5                   10                  15

Gly Arg Asn Gly Met Cys Val Asp Val Arg Asp Asp Asp Phe Arg Asp
            20                  25                  30

Gly Asn Gln Ile Gln Leu Trp Pro Ser Lys Ser Asn Asn Asp Pro Asn
        35                  40                  45

Gln Leu Trp Thr Ile Lys Arg Asp Gly Thr Ile Arg Ser Asn Gly Ser
    50                  55                  60

Cys Leu Thr Thr Tyr Gly Tyr Thr Ala Gly Val Tyr Val Met Ile Phe
65                  70                  75                  80

Asp Cys Asn Thr Ala Val Arg Glu Ala Thr Leu Trp Gln Ile Trp Gly
                85                  90                  95

Asn Gly Thr Ile Ile Asn Pro Arg Ser Asn Leu Val Leu Ala Ala Ser
            100                 105                 110

Ser Gly Ile Lys Gly Thr Thr Leu Thr Val Gln Thr Leu Asp Tyr Thr
        115                 120                 125

Leu Gly Gln Gly Trp Leu Ala Gly Asn Asp Thr Ala Pro Arg Glu Val
    130                 135                 140

Thr Ile Tyr Gly Phe Arg Asp Leu Cys Met Glu Ser Asn Gly Gly Ser
145                 150                 155                 160

Val Trp Val Glu Thr Cys Val Ser Ser Gln Lys Asn Gln Arg Trp Ala
                165                 170                 175

Leu Tyr Gly Asp Gly Ser Ile Arg Pro Lys Gln Asn Gln Asp Gln Cys
            180                 185                 190

Leu Thr Cys Gly Arg Asp Ser Val Ser Thr Val Ile Asn Ile Val Ser
        195                 200                 205

Cys Ser Ala Gly Ser Ser Gly Gln Arg Trp Val Phe Thr Asn Glu Gly
    210                 215                 220

Ala Ile Leu Asn Leu Lys Asn Gly Leu Ala Met Asp Val Ala Gln Ala
225                 230                 235                 240

Asn Pro Lys Leu Arg Arg Ile Ile Ile Tyr Pro Ala Thr Gly Lys Pro
                245                 250                 255

Asn Gln Met Trp Leu Pro Val Pro
            260

<210>  5
<211>  268
<212>  PRT
<213>  Artificial

<220>
<223>  EP0751221 recombinant Protein

<220>
<221>  misc_feature
<222>  (1)..(1)
<223>  Xaa can be Met or can be deleted

<400>  5

Xaa Asp Asp Val Thr Cys Ser Ala Ser Glu Pro Thr Val Arg Ile Val
1               5                   10                  15

Gly Arg Asn Gly Met Cys Val Asp Val Arg Asp Asp Asp Phe Arg Asp
            20                  25                  30

Gly Asn Gln Ile Gln Leu Trp Pro Ser Lys Ser Asn Asn Asp Pro Asn
        35                  40                  45

Gln Leu Trp Thr Ile Lys Arg Asp Gly Thr Ile Arg Ser Asn Gly Ser
    50                  55                  60

Cys Leu Thr Thr Tyr Gly Tyr Thr Ala Gly Val Tyr Val Met Ile Phe
65                  70                  75                  80

Asp Cys Asn Thr Ala Val Arg Glu Ala Thr Leu Trp Gln Ile Trp Gly
                85                  90                  95

Asn Gly Thr Ile Ile Asn Pro Arg Ser Asn Leu Val Leu Ala Ala Ser
            100                 105                 110

Ser Gly Ile Lys Gly Thr Thr Leu Thr Val Gln Thr Leu Asp Tyr Thr
        115                 120                 125

Leu Gly Gln Gly Trp Leu Ala Gly Asn Asp Thr Ala Pro Arg Glu Val
    130                 135                 140

Thr Ile Tyr Gly Phe Arg Asp Leu Cys Met Glu Ser Asn Gly Gly Ser
145                 150                 155                 160

Val Trp Val Glu Thr Cys Val Ser Ser Gln Lys Asn Gln Arg Trp Ala
                165                 170                 175

Leu Tyr Gly Asp Gly Ser Ile Arg Pro Lys Gln Asn Gln Asp Gln Cys
            180                 185                 190

Leu Thr Cys Gly Arg Asp Ser Val Ser Thr Val Ile Asn Ile Val Ser
        195                 200                 205

Cys Ser Ala Gly Ser Ser Gly Gln Arg Trp Val Phe Thr Asn Glu Gly
    210                 215                 220

Ala Ile Leu Asn Leu Lys Asn Gly Leu Ala Met Asp Val Ala Gln Ala
225                 230                 235                 240

Asn Pro Lys Leu Arg Arg Ile Ile Ile Tyr Pro Ala Thr Gly Lys Pro
                245                 250                 255

Asn Gln Met Trp Leu Pro Val Pro Gly Gly Tyr His
            260                 265

<210>  6
<211>  265
<212>  PRT
<213>  Artificial

<220>
<223>  EP1051495 recombinant Protein

<220>
<221>  misc_feature
<222>  (1)..(1)
<223>  Xaa can be Met or can be deleted

<220>

<221>  misc_feature
<222>  (19)..(19)
<223>  Xaa can be Asn or Ser

<220>
<221>  misc_feature
<222>  (22)..(22)
<223>  Xaa can be Cys or Arg

<220>
<221>  misc_feature
<222>  (57)..(57)
<223>  Xaa can be Gly or Asn

<220>
<221>  misc_feature
<222>  (96)..(96)
<223>  Xaa can be Gly or Asn

<220>
<221>  misc_feature
<222>  (158)..(158)
<223>  Xaa can be Gly or Gln
<220>
<221>  misc_feature
<222>  (167)..(167)
<223>  Xaa can be Val or Asp

<220>
<221>  misc_feature
<222>  (171)..(171)
<223>  Xaa can be Gln or Lys

<220>
<221>  misc_feature
<222>  (174)..(175)
<223>  Xaa can be Gly or can be deleted or can be Gly-Arg or Gly-Lys or Arg or
Lys

<220>
<221>  misc_feature
<222>  (196)..(196)
<223>  Xaa can be Cys or Val or Ser

<220>
<221>  misc_feature
<222>  (212)..(213)
<223>  Xaa can be Ala-Ala or Ala-Gly or Gly-Ala or Gly-Gly

<220>
<221>  misc_feature
<222>  (215)..(216)
<223>  Xaa can be Ser-Ser or Ser-Gly or Gly-Ser or Gly-Gly

<220>
<221>  misc_feature
<222>  (225)..(225)
<223>  Xaa can be Gly or Tyr

<220>
<221>  misc_feature
<222>  (232)..(236)
<223>  Xaa232 can be Asn, Ser, Thr or Lys, Xaa233 can be Ser or Gly, Xaa234 can
be Leu or Pro, Xaa235 can be Ala or Met, Xaa 236 can be Met or Val

<220>
<221>  misc_feature
<222>  (265)..(265)

<223>  Xaa can be Pro or Phe

<400>  6

Xaa Asp Asp Val Thr Cys Ser Ala Ser Glu Pro Thr Val Arg Ile Val
1               5                   10                  15

Gly Arg Xaa Gly Met Xaa Val Asp Val Arg Asp Asp Asp Phe His Asp
            20                  25                  30

Gly Asn Gln Ile Gln Leu Trp Pro Ser Lys Ser Asn Asn Asp Pro Asn
        35                  40                  45

Gln Leu Trp Thr Ile Lys Arg Asp Xaa Thr Ile Arg Ser Asn Gly Ser
    50                  55                  60

Cys Leu Thr Thr Tyr Gly Tyr Thr Ala Gly Val Tyr Val Met Ile Phe
65                  70                  75                  80

Asp Cys Asn Thr Ala Val Arg Glu Ala Thr Ile Trp Gln Ile Trp Xaa
                85                  90                  95

Asn Gly Thr Ile Ile Asn Pro Arg Ser Asn Leu Val Leu Ala Ala Ser
            100                 105                 110

Ser Gly Ile Lys Gly Thr Thr Leu Thr Val Gln Thr Leu Asp Tyr Thr
        115                 120                 125

Leu Gly Gln Gly Trp Leu Ala Gly Asn Asp Thr Ala Pro Arg Glu Val
    130                 135                 140

Thr Ile Tyr Gly Phe Arg Asp Leu Cys Met Glu Ser Asn Xaa Gly Ser
145                 150                 155                 160

Val Trp Val Glu Thr Cys Xaa Ser Ser Gln Xaa Asn Gln Xaa Xaa Trp
                165                 170                 175

Ala Leu Tyr Gly Asp Gly Ser Ile Arg Pro Lys Gln Asn Gln Asp Gln
            180                 185                 190

Cys Leu Thr Xaa Gly Arg Asp Ser Val Ser Thr Val Ile Asn Ile Val
        195                 200                 205

Ser Cys Ser Xaa Xaa Ser Xaa Xaa Gln Arg Trp Val Phe Thr Asn Glu
    210                 215                 220

Xaa Ala Ile Leu Asn Leu Lys Xaa Xaa Xaa Xaa Xaa Asp Val Ala Gln
225                 230                 235                 240

Ala Asn Pro Lys Leu Arg Arg Ile Ile Ile Tyr Pro Ala Thr Gly Lys
                245                 250                 255

Pro Asn Gln Met Trp Leu Pro Val Xaa
            260                 265

<210>  7
<211>  264
<212>  PRT
<213>  Artificial

<220>
<223>  EP1051495 recombinant Protein

<220>
<221>  misc_feature
<222>  (1)..(1)
<223>  Xaa can be Met or can be deleted

<400>  7

Xaa Asp Asp Val Thr Cys Ser Ala Ser Glu Pro Thr Val Arg Ile Val
1               5                   10                  15

Gly Arg Asn Gly Met Cys Val Asp Val Arg Asp Asp Asp Phe His Asp
            20                  25                  30

Gly Asn Gln Ile Gln Leu Trp Pro Ser Lys Ser Asn Asn Asp Pro Asn
        35                  40                  45

Gln Leu Trp Thr Ile Lys Arg Asp Gly Thr Ile Arg Ser Asn Gly Ser
    50                  55                  60

Cys Leu Thr Thr Tyr Gly Tyr Thr Ala Gly Val Tyr Val Met Ile Phe
65                  70                  75                  80

Asp Cys Asn Thr Ala Val Arg Glu Ala Thr Ile Trp Gln Ile Trp Gly
                85                  90                  95

Asn Gly Thr Ile Ile Asn Pro Arg Ser Asn Leu Val Leu Ala Ala Ser
            100                 105                 110

Ser Gly Ile Lys Gly Thr Thr Leu Thr Val Gln Thr Leu Asp Tyr Thr
        115                 120                 125

Leu Gly Gln Gly Trp Leu Ala Gly Asn Asp Thr Ala Pro Arg Glu Val
    130                 135                 140

Thr Ile Tyr Gly Phe Arg Asp Leu Cys Met Glu Ser Asn Gly Gly Ser
145                 150                 155                 160

Val Trp Val Glu Thr Cys Val Ser Ser Gln Gln Asn Gln Arg Trp Ala
                165                 170                 175

Leu Tyr Gly Asp Gly Ser Ile Arg Pro Lys Gln Asn Gln Asp Gln Cys
            180                 185                 190

Leu Thr Cys Gly Arg Asp Ser Val Ser Thr Val Ile Asn Ile Val Ser
        195                 200                 205

Cys Ser Ala Gly Ser Ser Gly Gln Arg Trp Val Phe Thr Asn Glu Gly
    210                 215                 220

Ala Ile Leu Asn Leu Lys Asn Gly Leu Ala Met Asp Val Ala Gln Ala
225                 230                 235                 240

Asn Pro Lys Leu Arg Arg Ile Ile Ile Tyr Pro Ala Thr Gly Lys Pro
                245                 250                 255

Asn Gln Met Trp Leu Pro Val Pro
            260

<210>  8
<211>  265
<212>  PRT
<213>  Artificial

<220>
<223>  EP1051495 recombinant Protein

<220>
<221>  misc_feature
<222>  (1)..(1)
<223>  Xaa can be Met or can be deleted

<400>  8

Xaa Asp Asp Val Thr Cys Ser Ala Ser Glu Pro Thr Val Arg Ile Val
1               5                   10                  15

Gly Arg Asn Gly Met Arg Val Asp Val Arg Asp Asp Asp Phe His Asp
            20                  25                  30

Gly Asn Gln Ile Gln Leu Trp Pro Ser Lys Ser Asn Asn Asp Pro Asn
        35                  40                  45

Gln Leu Trp Thr Ile Lys Arg Asp Gly Thr Ile Arg Ser Asn Gly Ser
    50                  55                  60

Cys Leu Thr Thr Tyr Gly Tyr Thr Ala Gly Val Tyr Val Met Ile Phe
65                  70                  75                  80

Asp Cys Asn Thr Ala Val Arg Glu Ala Thr Ile Trp Gln Ile Trp Asp
                85                  90                  95

Asn Gly Thr Ile Ile Asn Pro Arg Ser Asn Leu Val Leu Ala Ala Ser
            100                 105                 110

Ser Gly Ile Lys Gly Thr Thr Leu Thr Val Gln Thr Leu Asp Tyr Thr
        115                 120                 125

Leu Gly Gln Gly Trp Leu Ala Gly Asn Asp Thr Ala Pro Arg Glu Val
    130                 135                 140

Thr Ile Tyr Gly Phe Arg Asp Leu Cys Met Glu Ser Asn Gly Gly Ser
145                 150                 155                 160

Val Trp Val Glu Thr Cys Asp Ser Ser Gln Lys Asn Gln Gly Lys Trp
                165                 170                 175

Ala Leu Tyr Gly Asp Gly Ser Ile Arg Pro Lys Gln Asn Gln Asp Gln
            180                 185                 190

Cys Leu Thr Ser Gly Arg Asp Ser Val Ser Thr Val Ile Asn Ile Val
        195                 200                 205

Ser Cys Ser Gly Ala Ser Gly Ser Gln Arg Trp Val Phe Thr Asn Glu
    210                 215                 220

Gly Ala Ile Leu Asn Leu Lys Asn Gly Leu Ala Met Asp Val Ala Gln
225                 230                 235                 240

Ala Asn Pro Lys Leu Arg Arg Ile Ile Ile Tyr Pro Ala Thr Gly Lys
                245                 250                 255

Pro Asn Gln Met Trp Leu Pro Val Phe
            260                 265

<210>  9
<211>  265
<212>  PRT
<213>  Artificial

<220>
<223>  EP1051495 recombinant Protein

<220>
<221>  misc_feature
<222>  (1)..(1)
<223>  Xaa can be Met or can be deleted

<400>  9

Xaa Asp Asp Val Thr Cys Ser Ala Ser Glu Pro Thr Val Arg Ile Val
1               5                   10                  15

Gly Arg Ser Gly Met Arg Val Asp Val Arg Asp Asp Asp Phe His Asp
            20                  25                  30

Gly Asn Gln Ile Gln Leu Trp Pro Ser Lys Ser Asn Asn Asp Pro Asn
        35                  40                  45

Gln Leu Trp Thr Ile Lys Arg Asp Asn Thr Ile Arg Ser Asn Gly Ser
    50                  55                  60

Cys Leu Thr Thr Tyr Gly Tyr Thr Ala Gly Val Tyr Val Met Ile Phe
65                  70                  75                  80

Asp Cys Asn Thr Ala Val Arg Glu Ala Thr Ile Trp Gln Ile Trp Asp
                85                  90                  95

Asn Gly Thr Ile Ile Asn Pro Arg Ser Asn Leu Val Leu Ala Ala Ser
            100                 105                 110

Ser Gly Ile Lys Gly Thr Thr Leu Thr Val Gln Thr Leu Asp Tyr Thr
        115                 120                 125

Leu Gly Gln Gly Trp Leu Ala Gly Asn Asp Thr Ala Pro Arg Glu Val
    130                 135                 140

Thr Ile Tyr Gly Phe Arg Asp Leu Cys Met Glu Ser Asn Gln Gly Ser
145                 150                 155                 160

Val Trp Val Glu Thr Cys Asp Ser Ser Gln Lys Asn Gln Gly Lys Trp
                165                 170                 175

Ala Leu Tyr Gly Asp Gly Ser Ile Arg Pro Lys Gln Asn Gln Asp Gln
            180                 185                 190

Cys Leu Thr Val Gly Arg Asp Ser Val Ser Thr Val Ile Asn Ile Val
        195                 200                 205

Ser Cys Ser Gly Ala Ser Gly Ser Gln Arg Trp Val Phe Thr Asn Glu
    210                 215                 220

Tyr Ala Ile Leu Asn Leu Lys Ser Gly Leu Ala Met Asp Val Ala Gln
225                 230                 235                 240

Ala Asn Pro Lys Leu Arg Arg Ile Ile Ile Tyr Pro Ala Thr Gly Lys
                245                 250                 255

Pro Asn Gln Met Trp Leu Pro Val Phe
            260                 265


<210>  10
<211>  265
<212>  PRT
<213>  Artificial

<220>
<223>  EP1051495 recombinant Protein


<220>
<221>  misc_feature
<222>  (1)..(1)
<223>  Xaa can be Met or can be deleted

<400>  10

Xaa Asp Asp Val Thr Cys Ser Ala Ser Glu Pro Thr Val Arg Ile Val
1               5                   10                  15

Gly Arg Asn Gly Met Arg Val Asp Val Arg Asp Asp Asp Phe His Asp
            20                  25                  30

Gly Asn Gln Ile Gln Leu Trp Pro Ser Lys Ser Asn Asn Asp Pro Asn
        35                  40                  45

Gln Leu Trp Thr Ile Lys Arg Asp Gly Thr Ile Arg Ser Asn Gly Ser
    50                  55                  60

Cys Leu Thr Thr Tyr Gly Tyr Thr Ala Gly Val Tyr Val Met Ile Phe
65                  70                  75                  80

Asp Cys Asn Thr Ala Val Arg Glu Ala Thr Ile Trp Gln Ile Trp Asp
                85                  90                  95

Asn Gly Thr Ile Ile Asn Pro Arg Ser Asn Leu Val Leu Ala Ala Ser
            100                 105                 110

Ser Gly Ile Lys Gly Thr Thr Leu Thr Val Gln Thr Leu Asp Tyr Thr
        115                 120                 125

Leu Gly Gln Gly Trp Leu Ala Gly Asn Asp Thr Ala Pro Arg Glu Val
    130                 135                 140

Thr Ile Tyr Gly Phe Arg Asp Leu Cys Met Glu Ser Asn Gly Gly Ser
145                 150                 155                 160

Val Trp Val Glu Thr Cys Asp Ser Ser Gln Lys Asn Gln Gly Lys Trp
                165                 170                 175

Ala Leu Tyr Gly Asp Gly Ser Ile Arg Pro Lys Gln Asn Gln Asp Gln
            180                 185                 190

Cys Leu Thr Ser Gly Arg Asp Ser Val Ser Thr Val Ile Asn Ile Val
        195                 200                 205

Ser Cys Ser Gly Ala Ser Gly Ser Gln Arg Trp Val Phe Thr Asn Glu
    210                 215                 220

Gly Ala Ile Leu Asn Leu Lys Thr Gly Leu Ala Met Asp Val Ala Gln
225                 230                 235                 240

Ala Asn Pro Lys Leu Arg Arg Ile Ile Ile Tyr Pro Ala Thr Gly Lys
                245                 250                 255

Pro Asn Gln Met Trp Leu Pro Val Phe
            260                 265

<210>  11
<211>  265
<212>  PRT
<213>  Artificial

<220>
<223>  EP1051495 recombinant Protein


<220>
<221>  misc_feature
<222>  (1)..(1)
<223>  Xaa can be Met or can be deleted

<400>  11

Xaa Asp Asp Val Thr Cys Ser Ala Ser Glu Pro Thr Val Arg Ile Val
1               5                   10                  15


Gly Arg Asn Gly Met Arg Val Asp Val Arg Asp Asp Asp Phe His Asp
            20                  25                  30


Gly Asn Gln Ile Gln Leu Trp Pro Ser Lys Ser Asn Asn Asp Pro Asn
        35                  40                  45


Gln Leu Trp Thr Ile Lys Arg Asp Gly Thr Ile Arg Ser Asn Gly Ser
    50                  55                  60


Cys Leu Thr Thr Tyr Gly Tyr Thr Ala Gly Val Tyr Val Met Ile Phe
65                  70                  75                  80


Asp Cys Asn Thr Ala Val Arg Glu Ala Thr Ile Trp Gln Ile Trp Asp
                85                  90                  95


Asn Gly Thr Ile Ile Asn Pro Arg Ser Asn Leu Val Leu Ala Ala Ser
            100                 105                 110


Ser Gly Ile Lys Gly Thr Thr Leu Thr Val Gln Thr Leu Asp Tyr Thr
        115                 120                 125


Leu Gly Gln Gly Trp Leu Ala Gly Asn Asp Thr Ala Pro Arg Glu Val
    130                 135                 140


Thr Ile Tyr Gly Phe Arg Asp Leu Cys Met Glu Ser Asn Gly Gly Ser
145                 150                 155                 160


Val Trp Val Glu Thr Cys Asp Ser Ser Gln Lys Asn Gln Gly Lys Trp
                165                 170                 175


Ala Leu Tyr Gly Asp Gly Ser Ile Arg Pro Lys Gln Asn Gln Asp Gln
            180                 185                 190


Cys Leu Thr Ser Gly Arg Asp Ser Val Ser Thr Val Ile Asn Ile Val
        195                 200                 205

Ser Cys Ser Gly Ala Ser Gly Ser Gln Arg Trp Val Phe Thr Asn Glu
    210                 215                 220

Gly Ala Ile Leu Asn Leu Lys Lys Gly Pro Ala Met Asp Val Ala Gln
225                 230                 235                 240

Ala Asn Pro Lys Leu Arg Arg Ile Ile Ile Tyr Pro Ala Thr Gly Lys
                245                 250                 255

Pro Asn Gln Met Trp Leu Pro Val Phe
            260                 265

<210>  12
<211>  265
<212>  PRT
<213>  Artificial

<220>
<223>  EP1051495 recombinant Protein

<220>
<221>  misc_feature
<222>  (1)..(1)
<223>  Xaa can be Met or can be deleted

<400>  12

Xaa Asp Asp Val Thr Cys Ser Ala Ser Glu Pro Thr Val Arg Ile Val
1               5                   10                  15

Gly Arg Asn Gly Met Arg Val Asp Val Arg Asp Asp Asp Phe His Asp
            20                  25                  30

Gly Asn Gln Ile Gln Leu Trp Pro Ser Lys Ser Asn Asn Asp Pro Asn
        35                  40                  45

Gln Leu Trp Thr Ile Lys Arg Asp Gly Thr Ile Arg Ser Asn Gly Ser
    50                  55                  60

Cys Leu Thr Thr Tyr Gly Tyr Thr Ala Gly Val Tyr Val Met Ile Phe
65                  70                  75                  80

Asp Cys Asn Thr Ala Val Arg Glu Ala Thr Ile Trp Gln Ile Trp Asp
                85                  90                  95

Asn Gly Thr Ile Ile Asn Pro Arg Ser Asn Leu Val Leu Ala Ala Ser
            100                 105                 110

Ser Gly Ile Lys Gly Thr Thr Leu Thr Val Gln Thr Leu Asp Tyr Thr
        115                 120                 125

Leu Gly Gln Gly Trp Leu Ala Gly Asn Asp Thr Ala Pro Arg Glu Val
    130                 135                 140

Thr Ile Tyr Gly Phe Arg Asp Leu Cys Met Glu Ser Asn Gly Gly Ser
145                 150                 155                 160

Val Trp Val Glu Thr Cys Asp Ser Ser Gln Lys Asn Gln Gly Lys Trp
                165                 170                 175

Ala Leu Tyr Gly Asp Gly Ser Ile Arg Pro Lys Gln Asn Gln Asp Gln
            180                 185                 190

Cys Leu Thr Ser Gly Arg Asp Ser Val Ser Thr Val Ile Asn Ile Val
        195                 200                 205

Ser Cys Ser Gly Ala Ser Gly Ser Gln Arg Trp Val Phe Thr Asn Glu
    210                 215                 220

Gly Ala Ile Leu Asn Leu Lys Asn Ser Leu Met Val Asp Val Ala Gln
225                 230                 235                 240

Ala Asn Pro Lys Leu Arg Arg Ile Ile Ile Tyr Pro Ala Thr Gly Lys
                245                 250                 255

Pro Asn Gln Met Trp Leu Pro Val Phe
            260                 265